# EUROPEAN PATENT SPECIFICATION

(11) **EP 0 916 643 B1**
(45) Date of publication and mention of the grant of the patent: **19.12.2001**
(21) Application number: 98309235.4
(22) Date of filing: 11.11.1998
(51) Int. Cl.: C07C 67/08, C07C 69/54, C07C 67/62

(54) **A process for preparing alkyl (meth)acrylates**
Verfahren zum Herstellen von Alkyl (Meth)acrylaten
Procédé de préparation de (méth)acrylates d'alkyle

(30) Priority: 17.11.1997 US 66939 P
(43) Date of publication of application: 19.05.1999
(73) Proprietor: ROHM AND HAAS COMPANY, Philadelphia, Pennsylvania 19106-2399 (US)
(72) Inventor: Venter, Jeremia Jesaja, Hatfield, PA 19040 (US); Mirabelli, Mario Giuseppe Luciano, Horsham, PA 19044 (US)
(74) Representative: Buckley, Guy Julian

(56) References cited:
- EP-A- 0 080 023
- EP-A- 0 620 206
- EP-A- 0 733 617
- EP-A- 0 765 859

## Description

This invention relates to a process for preparing alkyl (meth)acrylates. Specifically, the process provides for the synthesis of alkyl (meth)acrylates, the hydrolysis of process impurities into starting materials, and the separation of reaction products and starting materials in one reactor.

Alkyl (meth)acrylates are important monomers in commercial polymerization processes. Conventionally, alkyl (meth)acrylates, such as butyl acrylate ("BA"), are commercially prepared by a direct esterification process. Typically, butanol ("BuOH") and acrylic acid ("AA") are reacted in the presence of an acid catalyst thereby yielding butyl acrylate and water. The direct esterification is generally run at elevated temperature and reduced pressure. During the reaction, impurities are formed such as dibutyl ether ("DBE"), butyl-β-butopy propionate ("BBBP"), butyl-β-hydroxy propionate ("BBHP"), butyl-acryloxypropionate ("BAOPA"), and acryloxypropionic acid ("AOPA"). These impurities, if not converted back to starting materials, result in lower yield.

Such impurities are usually removed from the reactor and treated to produce starting materials which can be reused. As a result, these processes are less efficient and require additional capital investment costs for separate reactors. Furthermore, conventional BA preparation processes operate at reduced pressure, necessitating a need for larger sized equipment. Consequently, there is a need for a more efficient, lower cost butyl acrylate process which converts process impurities back to starting materials, does so in the same reactor in which BA is produced and which can be operated at atmospheric pressure.

U.S. Patent No.5877345 discloses a process for preparing BA in which process impurities are hydrolyzed in separate reactors. In addition, the AA/BA separation is performed in a separate low pressure distillation column. Although the patent application addresses the issue of conversion of impurities to starting materials, it does not provide a BA process in which BA is prepared in a high water reaction medium which allows (1) operation under atmospheric pressure, (2) separation of AA/BA in the reactor, and (3) recovery of starting materials from process impurities in one unit.

European Patent Application No. 0080023 discloses a process for esterifying methacrylic acid containing from 5% to 60% by weight of water with a lower alkanol in the presence of sulfuric acid or an organic sulfonic acid, continuously distilling off a methacrylic ester/water azeotrope and separating the ester from the condensed distillate. A polymerisation inhibitor may be added to the reaction mixture, preferably hydroquinone or hydroquinone methyl ester.

The present invention discloses a process of preparing alkyl (meth)acrylates which converts process impurities back to starting materials and further reacts them in one reactor. The addition of water during the direct esterification reaction provides an alkyl (meth)acrylate preparation process which does not require reduced pressure and facilitates the recovery of starting materials from process impurities rendering yields greater than 100% possible. Furthermore, separation of the reaction product, such as BA, and the starting (meth)acrylic acid, such as AA, can also be effected in the reactor. Consequently, the present invention provides a process which is efficient and economical .

The present invention provides a process for the production of a C₁ to C₄ alkyl (meth)acrylate by (A) charging a reactor with a C₁ - C₄ alcohol, a (meth)acrylic acid, a strong acid catalyst, at least one inhibitor and at least 5% by weight water to form a reaction mixture, (B) reacting the reaction mixture to form a C₁ - C₄ alkyl (meth)acrylate and process impurities, ; and (C) separating the C₁ - C₄ alkyl (meth)acrylate and water formed during the reaction from the reaction mixture wherein the strong acid catalyst is selected from sulfuric acid, alkyl sulfonic acid and polymer supported alkyl sulfonic acid the at least one inhibitor is or includes 2,2,6,6-tetramethyl-1-piperidinyloxy or derivatives thereof and wherein the process impurities are hydrolysed in the reactor.

Preferably the present invention provides a process which includes: (A) charging a reactor with butanol, acrylic acid, a strong acid catalyst, and at least 5% by weight water to form a reaction mixture; (B) reacting the reaction mixture to form butyl acrylate and process impurities, wherein the process impurities are hydrolyzed in the reactor; and (C) separating the butyl acrylate and water formed during the reaction from the reaction mixture.

Preferably the present invention also provides a process which includes: (A) charging a reactor with butanol, acrylic acid, 3.5 to 15% by weight sulfuric acid, 6 to 18% by weight water and at least one inhibitor to form a reaction mixture, wherein the butanol and acrylic acid are charged to the reactor in an acrylic acid to butanol molar ratio of 1:1 to 1:1.7; (B) reacting the reaction mixture to form butyl acrylate and process impurities, wherein the process impurities are hydrolyzed in the reactor; and (C) separating the butyl acrylate and water formed during the reaction from the reaction mixture by azeotropic distillation.

As used herein, the term "(meth)acrylic" acid is meant to include both acrylic acid and methacrylic acid. In a like manner, the term "(meth)acrylate" is meant to include both acrylate and methacrylate.

As used herein, BuOH refers to n-butanol, i.e., 1-butanol and the term "butanol" includes within its scope all butanol isomers as well as mixtures thereof.

The term "alkyl" is meant to include branched chain, straight chain or cyclic alkyl groups. As used herein the terminology "(C₁-C₄)" or "(C₁-C₁₀)" means a group having from 1 to 4 or 1 to 10 carbon atoms per group.

As used herein, the terms "AA rich" or "BA rich" are understood to mean fractions or components where AA or BA is the major (greater than 50% by weight) organic component of the composition.

Throughout this specification and claims, unless otherwise indicated, references to percentages are by weight, all temperatures by degree centigrade and all pressures are atmospheric.

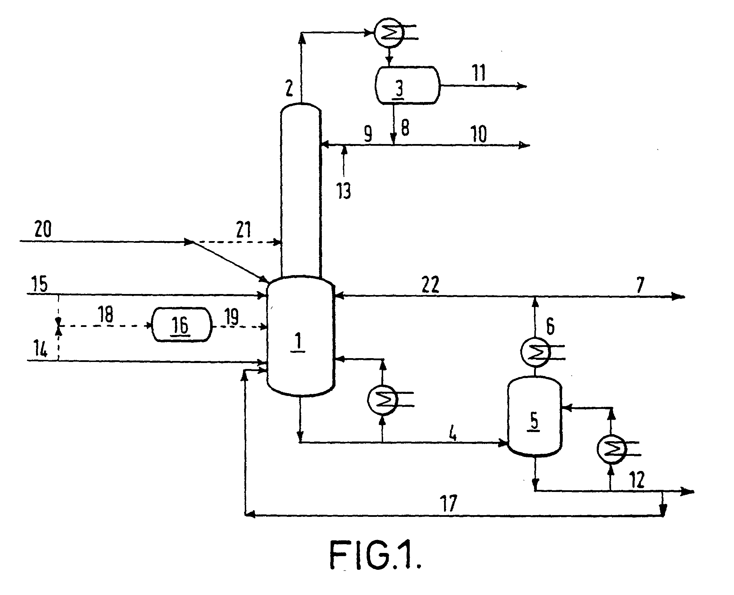

Figure 1 illustrates the equipment and the flow lines utilized in one embodiment of the process of the present invention, including the direct esterification/hydrolysis reactor **1** which is a stirred reactor having a distillation column on top of it; line **2**, which carries a vaporized distillate mixture, which includes BA, from **1** to a phase separator **3**, the phase separator **3** separates the vaporized distillate into a BA rich organic phase and an aqueous distillate phase; line **11**, which carries the BA rich organic distillate separated in **3** forward to a separation section; line **8** which carries the aqueous distillate separated in **3** to line **9** to be recycled to **1**, and to line **10** to carry it forward to be treated, generally to recover material from aqueous waste; line **4**, which carries the AA rich bottoms from **1** to bleed stripper **5**, which is the cracking reactor; line 6, which carries the distillate, including recovered BuOH and AA from **5** to be recycled to **1** through line **22**, and to line **7** which carries the distillate from **5** forward to be treated, generally as waste; line **12**, which carries the bottoms from **5** forward to be treated, generally as waste and optionally to line **17**, which recycles bottoms from **5** to **1**; line **13**, which may feed inhibitor to the reactor; line **14**, which feeds catalyst to the reactor; line **15**, which feeds fresh AA and BuOH to the reactor; an optional plug flow reactor **16**; an optional line **18** for feeding AA, BuOH, and catalyst to **16**; an optional line **19** for taking the material from **16** to **1**; line **20**, which carries the BuOH, BA, and AA recovered in the separation section from lines **10** and **11** back to the reactor **1**; and optional line **21** which returns recovered material to an alternative feed location in reactor **1**.

As recited above, in step (A) of the present invention C₁ - C₄ alcohol, a (meth)acrylic acid, a strong acid catalyst, and water are charged to a reactor to form a reaction mixture.

Generally, the C₁ - C₄ alcohol is a branched or straight chain alkanol having 1 to 4 carbon atoms or mixture thereof. Specific examples include, but are not limited to, methanol, ethanol, n-propanol, isopropanol, n-butanol, 2-butanol, tert-butanol or mixtures thereof. Furthermore, it is contemplated that the C₁ - C₄ alcohol may be substituted, for example, with halogen, hydroxide, alkoxide, cyano, nitro, etc. In one embodiment, the alcohol is butanol. In a preferred embodiment, the alcohol is n-butanol.

Also present in the reaction mixture is (meth)acrylic acid or substituted meth(acrylic) acid substituted with, for example, with halogen, hydroxide, alkoxide, cyano, nitro, etc.. In one embodiment, acrylic acid or methacrylic acid or a mixture thereof is present. In a preferred embodiment, the unsaturated acid is acrylic acid. The (meth)acrylic acid and alcohol are present in a molar ratio of 1:1 to 1:1.7, preferably 1:1.1 to 1:1.6, more preferably 1:1.25 to 1:1.45. It is also contemplated that other unsaturated acids such as crotonic acid, cinnamic acid, maleic acid, fumaric acid, etc., which can participate in a transesterification reaction with an alcohol may be utilized in the process of the present invention.

A strong acid catalyst is also present in the reaction mixture. Suitable examples of such an acid catalyst include, but are not limited to, sulfuric acid, methane sulfonic acid, benzene sulfonic acid, p-toluene sulfonic acid, mixtures thereof, or a polymer supported alkyl sulfonic acids such as AMBERLYST™ 15 resin or NAFION-H™ resin. Generally, the alkyl sulfonic acid is a C₁ to C₁₀ alkyl sulfonic acid. In one embodiment, the strong acid catalyst is a sulfur containing acid or sulfur containing polymer supported acid. In a preferred embodiment, the strong acid catalyst is sulfuric acid. The concentration of strong acid strong acid catalyst by total weight of the reaction mixture in the direct esterification/hydrolysis reactor is typically 3.5 to 15% by weight, preferably 3 to 12% by weight, and more preferably 5 to 8% by weight.

Water is also present in the reaction mixture provided in step (A). Generally, any water, such as tap water, distilled water or deionized water, suitable for use in a direct transesterification reaction, may be used. Furthermore, at least some of the water provided may be recycled water of reaction which has been removed during separation of the reaction product from the starting materials. The addition of water provides a water reaction medium in the reactor which enables operation under atmospheric conditions and the hydrolysis of reaction byproducts to recover starting materials as well as separation of reaction products from starting materials in one reactor.

Typically, from 0.001 to 1.0%, preferably 0.001 to 0.5%, and more preferably 0.001 to 0.1% by total weight of reaction mixture of the at least one polymerisation inhibitor is present during the direct esterification process. Suitable inhibitors include,besides 2,2,6,6-tetramethyl-1-piperidinyloxy, derivatives including but not limited to 4-methacryloyloxy-2,2,6,6-tetramethyl piperidinyl free radical and 4-hydroxy-2,2,6,6-tetramethyl N-hydroxy piperidine. In a preferred embodiment, the at least one inhibitor is 2,2,6,6-tetramethyl-1-piperidinyloxy. In another embodiment, 2,2,6,6-tetramethyl-1-piperidinyloxy and another inhibitor such as, the methyl ether of hydroquinone are used.
Other inhibitors which may be used are hydroquinone, the mono-methyl ether of hydroquinone, butylated hydroxy anisole, naphthoquinone, anthranil, and combinations thereof.

In one embodiment, the alcohol is butanol, the (meth)acrylic acid is acrylic acid, the acid catalyst is sulfuric acid and the inhibitor is 2,2,6,6-tetramethyl-1-piperidinyloxy. In a further embodiment the water is present at from 6 to 18% by total weight of reaction mixture and strong acid catalyst is present at 3.5 to 15% by total weight of reaction mixture.

As recited above, the present invention utilizes one reactor wherein direct esterification of AA and BuOH; hydrolysis of reaction byproducts including BBBP, BBHP, and BAOPA; and separation of BA and AA are achieved. Generally, any reactor suitable or adaptable for a process wherein a direct esterification reaction, hydrolysis of reaction byproducts formed during the transesterification reaction, and separation of reaction products from starting materials occurs in the reactor may be used. In one embodiment, the reactor may be a stirred tank equipped with a distillation column. In a preferred embodiment, the distillation column may be situated directly on top of the reactor (as in Figure 1) and may be a fractional distillation column. Generally, the distillation column contains from 20 to 100 trays. It is preferred that the column contains from 20 to 70 trays. It is more preferred that the column contains from 40 to 50 trays. The column has means for feeding AA, BuOH, a strong acid catalyst, water, and at least one inhibitor. The reactor also has means for removing the bottoms.

In step (B) the reaction mixture is reacted to form a C₁ - C₄ alkyl (meth)acrylate and reaction byproducts, while reaction byproducts formed during the reaction are hydrolyzed in the same reactor.

The direct esterification reaction may be run by feeding AA and BuOH through lines **15, 20,** and **22** to the direct esterification/hydrolysis reactor **1** in an AA to BuOH molar ratio ranging from 1:1 to 1:1.7, preferably 1:1.25 to 1:1.45. The AA and BuOH may also be fed along with sulfuric acid to a plug flow reactor **16**, and then to the direct esterification/hydrolysis reactor **1**. Inhibitor , if used, is fed into the reactor using line **13**. The AA, BuOH, inhibitor, strong acid catalyst, and water form a reaction mixture in the direct esterification/hydrolysis reactor. The AA and BuOH are reacted to a conversion on AA of from 50 to 95%, preferably 60 to 95%, more preferably 70 to 95%.

During the direct esterification reaction, the reactor must have at least 5% by total weight of reaction mixture of water for efficient hydrolysis operation. Preferably, the reactor has from 6 to 18% by weight water during the direct esterification reaction. More preferably, the reactor has from 8 to 12% by weight water during the direct esterification reaction. Water content may be maintained by returning the condensed and separated aqueous distillate from the reactor through line **9** back to the reactor. Water may also be added through any of the feed lines as is necessary. The water in the reactor hydrolyzes reaction byproducts formed during the reaction. Specific examples of hydrolysis reactions which occur include, but are not limited to, reactions where BBBP is hydrolyzed to 2 BuOH and 1 AA, BBHP is hydrolyzed to 1 BuOH and 1 AA, and BAOPA is hydrolyzed to 1 BuOH and 2 AA.

The direct esterification reaction and hydrolysis are run at a temperature of from 100°C to 140°C, preferably 105°C to 135°C, and more preferably 115°C to 130°C. The direct esterification reaction and hydrolysis are run at pressures from 100 mm Hg to 760 mm Hg. Atmospheric pressure is preferred. The residence time in the direct esterification/hydrolysis reactor is typically from 0.5 to 5 hours, preferably from 1 to 4 hours, and more preferably from 2 to 3 hours.

In step (C), the C₁ - C₄ alkyl (meth)acrylate and water formed during the reaction of the alcohol with the (meth)acrylic acid are separated from the reaction mixture by methods known in the art such as distillation, phase separation, etc. In a preferred embodiment, the C₁ - C₄ alkyl (meth)acrylate and water formed during the reaction are separated from the reaction mixture by azeotropic distillation. In a more preferred embodiment, the C₁ - C₄ alkyl (meth)acrylate is azeotropically distilled with water (aqueous reflux) and BuOH under the conditions described above. Accordingly, the water added to the reaction medium as well as water produced from the transesterification reaction of AA and BuOH provide an aqueous medium which enhances separation of AA and BA in the reactor. The distillate may then be taken through line **2** to a phase separator **3**. In the phase separator, an organic phase which is BA rich and contains BuOH, and an aqueous phase which contains water and AA separate. The organic phase may be taken through line **11** to a separation section, wherein pure BA is obtained. BuOH may be recovered from the separation section and recycled. Part of the aqueous phase is taken through line **8** to line **9** to be recycled to the reactor to maintain the appropriate amount of water in the reactor. The rest of the aqueous phase is taken through line **8** to line **10** to carry it forward to be recovered and treated, generally as waste.

The bottoms of the direct esterification reactor contain strong acid catalyst, BA, AA, BuOH, AOPA, BBPA, and BHPA. A bleed stripper **5** may be utilized to crack acryloxy proprionic acid ("AOPA"), the dimer of AA; beta-n-butoxy propionic acid ("BBPA"), and beta-hydroxy propionic acid ("BHPA"). Accordingly, the bottoms may be taken through line **4** to bleed stripper 5 (the cracking reactor), where AOPA is cracked to 2 AA; BBPA is cracked to 1 BuOH and 1 AA; and BHPA is cracked to 1 AA. The cracking reactor may be a continuous stirred tank reactor. Where the cracking reactor is incorporated in the process, the liquid in the cracking reactor is maintained at from 5 to 25% by weight strong acid, preferably sulfuric acid. The cracking reactor is operated at a temperature ranging from 90 to 140°C, preferably from 110 to 140°C. The cracking reactor is operated at a pressure ranging from 20 to 200 mm Hg, although higher pressures, up to 800 mm Hg may be used. The residence time in the cracking reactor is typically from 0.5 to 10 hours, preferably 0.5 to 6 hours, more preferably 0.5 to 3 hours.

Part of the BA, AA, BuOH and water generated in the cracking reactor may be taken overhead and recycled to the direct esterification/hydrolysis reactor through line **6** to line **22**. The rest of the BA, AA, BuOH and water generated in the cracking reactor may be taken overhead through line **6** to line **7** to be carried forward to be treated, generally as waste. The bottoms of the cracking reactor may be taken through line **13** to be carried forward to be treated, generally as waste, or may be recycled to the direct esterification/hydrolysis reactor **1** through line **17**.

Abbreviations used throughout this application are:

| | |
|---|---|
| % = percent | °C = degrees Centigrade |
| BA = butyl acrylate | mm = millimeters |
| ml = milliliters | ml/min = milliliters per minute |
| AA = acrylic acid | BuOH = butanol |
| AOPA = acryloxypropionic acid | BBPA = beta-n-butoxy propionic acid |
| BHPA = beta-hydroxy propionic acid | BBBP = butyl-β-butoxy propionate |
| BBHP = butyl-β-hydroxy propionate | BAOPA = butyl-acryloxypropionate |
| cm = centimeters | Hg = Mercury |
| g/hr = grams per hour | |

The following examples illustrate the process of the present invention.

Materials: AA, BA, and BuOH were obtained from plant production streams. The inhibitors used are commercially available.

Analyses: Standard methods were used for determination of water, monomer, BuOH, and residual impurities. AOPA, BBBP, BBHP, and BAOPA levels were determined by gas/liquid chromatography using flame ionization detection. Sulfuric acid determinations were obtained using a pH probe and alcoholic tetrabutylammonium hydroxide titrant.

### Example 1

### Preparation of Butyl Acrylate

A direct esterification/hydrolysis reactor was set up using a 5,000 ml round bottom flask connected to a multi-tube Hastalloy C-276 steam jacketed reboiler. A 45 tray, 5.08 cm Oldershaw fractional distillation column was situated directly on top of the glass reactor, and was considered to be part of the reactor. An overhead system of two standard glass, water cooled condensers connected in series was connected to the fractional distillation column. A 2,000 ml glass fraction cutter was connected to the second condenser. A bleed stripper (cracking reactor) was set up using a 500 ml flask equipped with an electric heating mantle, temperature controllers, a stirrer and a water cooled distillation head having a take-off port leading to a 125 ml fraction cutter. A peristaltic pump was provided for pumping the bottoms stream directly from the reboiler. A graduated cylinder equipped with a Teflon stopcock for easy sample removal was used for collecting the bottoms stream.

AA, BuOH, and sulfuric acid were fed to the direct esterification reactor. The direct esterification reactor was set to 128°C and 760 mm Hg. The bleed stripper was set to 130°C and 35 mm Hg with 26 % by weight sulfuric acid present. The inhibitor pump was then turned on to pump a solution of 0.25% 2,2,6,6-tetramethyl-1-piperidinyloxy and 0.018% methyl ether of hydroquinone in BA. Once the top trays of the fractional distillation column were wetted with inhibitor solution, steam was introduced to the reboiler. When the reboiler reached the desired temperature and distillate was observed in the overhead system, the following were pumped into the reactor: 3.7 g/hr inhibitor via positive displacement FMI piston pump, 234.4 g/hr total AA (including recycle) via positive displacement FMI piston pump, 382.5 g/hr total BuOH (including recycle) via positive displacement FMI piston pump, and 4.5 g/hr sulfuric acid via a peristaltic pump utilizing appropriate tubing compatible with strong mineral acid. A portion of the water recovered from overhead was fed back to the reactor for a reflux feed rate of 461.8 g/hr. The residence time in the reactor was 180 minutes. Distillate and bottoms streams were collected hourly and analyzed for strong acid, AA, and water to determine when steady state had been achieved. Once steady state was achieved, all process streams were thoroughly analyzed.

Once distillate was observed in the bleed stripper water cooled distillation head, the feed pumps were turned on and distillate and bottoms streams were collected. To the bleed stripper was pumped 66.3 g/hr bottoms from the direct esterification reactor. The bottoms stream was collected via an overflow system in which the overflow point was set at a particular volume, which represented the desired residence time. Once steady state was achieved, the distillate stream was thoroughly analyzed. The residence time in the bleed stripper was 235 minutes. The distillate recovered overhead was pumped back to the direct esterification reactor at a rate of 47.1 g/hr. The process had a yield of 108% of BA based on AA. The process had a BA yield of 95% based on BuOH. Process yields in excess of 100% are possible because BA can be recovered from AOPA present in the AA feed.

### Example 2

### Preparation of Butyl Acrylate with Plug Flow Reactor Utilization

In this example, a plug flow reactor was inserted before the direct esterification reactor. The rest of the equipment and procedures were the same as in Example 1. The direct esterification reactor was set to 115°C and 760 mm Hg. To the plug flow reactor was pumped: 219 g/hr AA, 201.3 g/hr BuOH, and 2.2 g/hr sulfuric acid. To the direct esterification reactor was pumped 3.6 g/hr inhibitor, 422.5 g/hr effluent from the plug flow reactor, and 2 g/hr sulfuric acid. A portion of the water recovered from overhead was pumped back to the reactor for a reflux rate of 477.7 g/hr. The residence time in the reactor was 180 minutes. The bleed stripper was operated at 130°C and 35 mm Hg with 17.5 % by weight sulfuric acid present. To the bleed stripper was pumped 96.8 g/hr bottoms from the direct esterification reactor. The residence time in the bleed stripper was 195 minutes. The distillate recovered overhead was pumped back to the direct esterification reactor at a rate of 72.1 g/hr. The process had a BA yield of 98% based on AA. The process had a BA yield of 100% based on BuOH.

### Example 3

### Preparation of Butyl Acrylate with Cracking Reactor Bottoms Recycle

In this example, the bottoms from the cracking reactor were recycled to the direct esterification reactor. The rest of the equipment and procedures were the same as in Example 1. The direct esterification reactor was set to 130°C and 760 mm Hg. To the direct esterification reactor was pumped 3.8 g/hr inhibitor, 230.8 g/hr AA, 377.4 g/hr BuOH, and 3.2 g/hr sulfuric acid. A portion of the water recovered from overhead was pumped back to the reactor for a reflux rate of 468.7 g/hr. The residence time in the reactor was 154 minutes. The bleed stripper was operated at 130°C and 35 mm Hg with 22.2% by weight sulfuric acid present. To the bleed stripper was pumped 65 g/hr bottoms from the direct esterification reactor. The residence time in the bleed stripper was 195 minutes. The distillate recovered overhead was pumped back to the direct esterification reactor at a rate of 36.4 g/hr. The bottoms of the bleed stripper were recycled to the direct esterification reactor at a rate of 10.5 g/hr. The process had a BA yield of 98% based on AA. The process had a BA yield of 100% based on BuOH.

### Example 4

### Preparation of Butyl Acrylate Using Methane Sulfonic Acid Catalyst

In this example, methane sulfonic acid was substituted for sulfuric acid as the catalyst. The rest of the equipment and procedures were the same as in Example 1. The direct esterification reactor was set to 119°C and 760 mm Hg. To the direct esterification reactor was pumped 3.8 g/hr inhibitor, 212.1 g/hr AA, 383.9 g/hr BuOH, and 5.2 g/hr methane sulfonic acid. A portion of the water recovered from overhead was pumped back to the reactor for a reflux rate of 470.5 g/hr. The residence time in the reactor was 131 minutes. The bleed stripper was operated at 130°C and 35 mm Hg with 28% by weight methane sulfonic acid present. To the bleed stripper was pumped 73 g/hr bottoms from the direct esterification reactor. The residence time in the bleed stripper was 278 minutes. The distillate recovered overhead was pumped back to the direct esterification reactor at a rate of 57.3 g/hr. The process had a BA yield of 98% based on AA. The process had a BA yield of 100% based on BuOH.

The above examples demonstrate that the process of this invention is effective at producing alkyl (meth)acrylates in high yields by direct esterification efficiently and economically. That is, the process is run in a high aqueous medium wherein:
(1) reaction pressures can be atmospheric and need not be reduced pressures;
(2) AA and BA, i.e. starting materials and reaction product are separated in the reactor; and
(3) the transesterification reaction and byproduct hydrolysis reaction occur in the same reactor.

Accordingly, the need for additional and separate equipment and process steps (for byproduct hydrolysis and/or reaction product/starting material separation) and/or larger equipment (because of lower reaction pressures) is eliminated.

## Claims

1. A process for the production of a C₁ to C₄ alkyl (meth)acrylate by (A) charging a reactor with a C₁ - C₄ alcohol, a (meth)acrylic acid, a strong acid catalyst, at least one inhibitor and at least 5% by weight water to form a reaction mixture, (B) reacting the reaction mixture to form a C₁ - C₄ alkyl (meth)acrylate and process impurities,; and (C) separating the C₁ - C₄ alkyl (meth)acrylate and water formed during the reaction from the reaction mixture wherein the strong acid catalyst is selected from sulfuric acid, alkyl sulfonic acid and polymer supported alkyl sulfonic acid and the at least one inhibitor is or includes 2,2,6,6-tetramethyl-1-piperidinyloxy or derivatives thereof and wherein the process impurities are hydrolysed in the reactor.

2. The process according to Claim 1 wherein the inhibitor is or includes 4-methacryloyloxy-2,2,6,6-tetramethyl piperidinyl free radical, 4-hydroxy-2,2,6,6-tetramethyl N-hydroxy piperidine, 2,2,6,6-tetramethyl-1-piperidinyloxy and combinations thereof.

3. The process according to Claim 1 wherein the inhibitor is or includes 2,2,6,6-tetramethyl-1-piperidinyloxy.

4. The process according to Claim 1 wherein the separation of the C₁ - C₄ alkyl (meth)acrylate and water from the reaction mixture is achieved by azeotropic distillation directly from the reaction mixture.

5. The process according to Claim 4 wherein the azeotropic distillation is carried out in the reactor using aqueous reflux in a distillation column having from 20 to 100 trays.

6. The process according to Claim 5 wherein the azeotropic distillation is carried out at atmospheric pressure.

7. The process according to Claim 1 wherein the C₁ - C₄ alcohol is butanol, the (meth)acrylic acid is acrylic acid, the strong acid catalyst is sulfuric acid, and the inhibitor is 2,2,6,6-tetramethyl-1-piperidinyloxy.

8. The process according to Claim 7 wherein the butanol and the acrylic acid are charged to the reactor in an acrylic acid to butanol molar ratio of 1:1 to 1:1.7.

9. The process according to Claim 1 wherein the water is present at from 6 to 18% by weight and the strong acid catalys is present at from 3.5 to 15% by weight.

10. A reaction mixture comprising acrylic acid, butanol, from 3.5% to 15% by weight sulphuric acid, from 6% to 18% by weight water and from 0.001% to 1.0% by weight 2,2,6,6-tetramethyl-1-piperidinyloxy.

## Patentansprüche

1. Ein Verfahren zur Herstellung eines C₁-C₄-Alkyl(Meth)acrylats durch (A) Beladen eines Reaktionsgefäßes mit einem C₁-C₄-Alkohol, einer (Meth)acrylsäure, einem starken Säurekatalysator, mindestens einem Inhibitor und mindestens 5 Gew.-% Wasser, um ein Reaktionsgemisch zu bilden, (B) Umsetzen des Reaktionsgemisches, um ein C₁-C₄-Alkyl(meth)acrylat und verfahrensbedingte Verunreinigungen (process impurities) zu bilden; und (C) Trennen des C₁-C₄-Alkyl(meth)acrylats und des während der Reaktion gebildeten Wassers von dem Reaktionsgemisch, wobei der starke Säurekatalysator ausgewählt ist aus Schwefelsäure, Alkylsulfonsäure und Polymerträgeralkylsulfonsäure und wobei der mindestens eine Inhibitor 2,2,6,6-Tetramethyl-1-piperidinyloxy oder Derivate davon ist oder einschließt und wobei die verfahrensbedingten Verunreinigungen in dem Reaktionsgefäß hydrolysiert werden.

2. Das Verfahren nach Anspruch 1, worin der Inhibitor 4-Methacryloyloxy-2,2,6,6-Tetramethylpiperidinyl-Radikal, 4-Hydroxy-2,2,6,6-tetramethyl-N-hydroxypiperidin, 2,2,6,6-Tetramethyl-1-piperidinyloxy und Kombinationen davon ist oder einschließt.

3. Das Verfahren nach Anspruch 1, worin der Inhibitor 2,2,6,6-Tetramethyl-1-piperidinyloxy ist oder einschließt.

4. Das Verfarhen nach Anspruch 1, worin die Trennung von C₁-C₄-Alkyl(meth)acrylat und Wasser von dem Reaktionsgemisch durch azeotrope Destillation unmilelbar aus dem Reaktonsgemisch erfolgt.

5. Das Verfahren nach Anspruch 4, wobei die azeotrope Destillation in dem Reaktionsgemisch unter Einsatz von wäßrigem Rückfluß in einer Destillationssäule mit 20 bis 100 Böden (trays) erfolgt.

6. Das Verfahren nach Anspruch 5, wobei die azeotrope Destillation bei atmospherischem Druck durchgeführt wird.

7. Das Verfahren nach Anspruch 1, wobei der C₁-C₄-Alkohol Butanol ist, die (Meth)acrylsäure Acrylsäure ist, der starke Säurekatalysator Schwefelsäure ist und der Inhibitor 2,2,6,6-Tetramethyl-1-piperidinyloxy ist.

8. Das Verfahren nach Anspruch 7, wobei der Butanol und die Acrylsäure in das Reaktionsgefäß eingespeist werden in einem molaren Verhältnis von Acrylsäure zu Butanol von 1:1 bis 1:1,7.

9. Das Verfahren nach Anspruch 1, wobei das Wasser von 6 bis 18 Gew.-% ausmacht und der starke Säurekatalysator von 3,5 bis 15 Gew.-% ausmacht.

10. Eine Reaktionsgemisch, umfassend Acrylsäure, Butanol, von 3,5 Gew.-% bis 15 Gew.-% Schwefelsäure, von 6 Gew.-% bis 18 Gew.-% Wasser und von 0,001 Gew.-% bis 1,0 Gew.-% 2,2,6,6-Tetramethyl-1-Piperidinyloxy.

## Revendications

1. Procédé de production d'un (méth)acrylate d'alkyle en C₁-C₄, par (A) introduction, dans un réacteur, d'un alcool en C₁-C₄, d'un acide (méth)acrylique, d'un catalyseur acide fort, d'au moins un inhibiteur et d'au moins 5 % en poids d'eau pour former un mélange réactionnel ; (B) réaction du mélange réactionnel pour former un (méth)acrylate d'alkyle en C₁-C₄ et des impuretés du procédé ; et (C) séparation, d'avec le mélange réactionnel, du (méth)acrylate d'alkyle en C₁-C₄ et de l'eau formée au cours de la réaction, où le catalyseur acide fort est choisi parmi l'acide sulfurique, les acides alkylsulfoniques et les acides alkylsulfoniques supportés par un polymère, et le ou les inhibiteurs représentent ou comprennent le 2,2,6,6-tétraméthyl-1-pipéridinyloxy ou ses dérivés, et où les impuretés du procédé sont hydrolysées dans le réacteur.

2. Procédé selon la revendication 1, dans lequel l'inhibiteur représente ou comprend le radical libre 4-méthacryloyloxy-2,2,6,6-térraméthyl-pipéridinyle, la 4-hydroxy-2,2,6,6-tétraméthyl-N-hydroxypipéridine, le 2,2,6,6-tétraméthyl-1-pipéridinyloxy, et leurs combinaisons.

3. Procédé selon la revendication 1, dans lequel l'inhibiteur représente ou comprend le 2,2,6,6-tétraméthyl-1-pipéridinyloxy.

4. Procédé selon la revendication 1, dans lequel la séparation, d'avec le mélange réactionnel, du (méth)acrylate d'alkyle en C₁-C₄ et de l'eau est réalisée par distillation azéotropique, directement à partir du mélange réactionnel.

5. Procédé selon la revendication 4, dans lequel la distillation azéotropique est mise en oeuvre dans le réacteur par utilisation d'un reflux aqueux dans une colonne de distillation ayant de 20 à 100 plateaux.

6. Procédé selon la revendication 5, dans lequel la distillation azéotropique est mise en oeuvre sous la pression atmosphérique.

7. Procédé selon la revendication 1, dans lequel l'alcool en C₁-C₄ est le butanol, l'acide (méth)acrylique est l'acide acrylique, le catalyseur acide fort est l'acide sulfurique, et l'inhibiteur est le 2,2,6,6-tétraméthyl-1-pipéridinyloxy.

8. Procédé selon la revendication 7, dans lequel le butanol et l'acide acrylique sont introduits dans le réacteur selon un rapport en moles de l'acide acrylique au butanol de 1:1 à 1:1,7.

9. Procédé selon la revendication 1, dans lequel l'eau est présente en une quantité de 6 à 18 % en poids, et le catalyseur acide fort est présent en une quantité de 3,5 à 15 % en poids.

10. Mélange réactionnel comprenant de l'acide acrylique, du butanol, de 3,5 à 15 % en poids d'acide sulfurique, de 6 à 18 % en poids d'eau et de 0,001 à 1,0 % en poids de 2,2,6,6-tétraméthyl-1-pipéridinyloxy.
